# EUROPEAN PATENT SPECIFICATION

(11) **EP 2 424 462 B1**
(45) Date of publication and mention of the grant of the patent: **26.03.2014**
(21) Application number: 09779358.2
(22) Date of filing: 27.04.2009
(51) Int. Cl.: A61F 2/06, A61F 5/00, A61M 31/00, A61M 37/00, A61F 2/04, A61F 2/00

(54) **AN ADJUSTABLE SHAPE CHANGEABLE SURGICAL IMPLANT SYSTEM**
VERSTELLBARES FORMVERÄNDERBARES CHIRURGISCHES IMPLANTATIONSSYSTEM
SYSTÈME D'IMPLANT CHIRURGICAL À CHANGEMENT DE FORME AJUSTABLE

(43) Date of publication of application: 07.03.2012
(73) Proprietor: Ethicon Endo-Surgery, Inc., Cincinnati, Ohio 45242-2839 (US)
(72) Inventor: BALEK, Stephen, I-00124 Casal Palocco Roma (IT)
(74) Representative: Leihkauf, Steffen Falk
(86) International application number: PCT/EP2009/055037
(87) International publication number: WO 2010/124714

(56) References cited:
- WO-A-00/64385
- WO-A-2008/023160
- US-A1- 2009 053 281
- US-A1- 2009 088 846

## Description

The present invention relates, in general, to devices for surgically modifying organs and vessels and more particularly to shape changeable surgical implants which are deployed inside a patient's body in order to temporarily or definitively influence the shape and/or structure of organs. Examples for known shape changeable surgical implants are:
- gastric bands which can be fastened around a patients stomach to form a tightening ring which can be tightened or enlarged by mechanically pumping or discharging a saline solution in a dilating receptacle thereof,
- fecal incontinence devices which can be fastened around a patient's rectum and mechanically tightened to control defecation,
- urinary incontinence devices which can be fastened around a patient's urinary tract and mechanically tightened to control urination,
- bone stabilizing devices like adjustable screw connectable stabilizing and shape correction structures,
- temporary anastomosis devices or similar organ joining devices which can be implanted in the patient's body to temporarily hold adjoining physiological structures in tension or in compression.

Notwithstanding the relatively satisfying clinical results of the known shape changeable surgical implants, their adjustment requires still a relatively invasive intervention which can only be performed by a surgeon, e.g. the needle injection or withdrawal of saline solution in the known gastric bands or a surgical adjustment of bone stabilizing structures, incontinence devices and anastomosis devices, so that such adjustments are not allays arbitrarily often repeatable and, hence, the shape of the known implants is not always perfectly and timely adapted to the clinical conditions of the patients.

WO2008/023160A describes an endoluminal prosthesis comprising an agent that promotes swelling on exposure to contact with body fluid

The object of the present invention is therefore to provide an adjustable shape changeable surgical implant system which obviates at least part of the drawbacks of the known devices. A particular object of the present invention is to provide an adjustable shape changeable surgical implant system which enables a non invasive shape adjustment of the implant without involvement of a surgeon and which can be done by the patient himself.

A further object of the present invention is to provide an adjustable shape changeable surgical implant system allowing for nearly unlimited and quick shape adjustments.

A further object of the present invention is to provide an adjustable shape changeable surgical implant system allowing for implant shape adjustment through oral administration of a substance.

A yet further object of the present invention is to provide a shape changeable gastric band which can be enlarged and tightened without surgical interventions.

A yet further object of the present invention is to provide a shape changeable fecal incontinence device which can be fastened around a patient's rectum and enlarged and tightened without surgical interventions to control defecation,

A yet further object of the present invention is to provide a shape changeable urinary incontinence device which can be fastened around a patient's urinary tract and enlarged and tightened without surgical interventions to control urination,

A yet further object of the present invention is to provide a shape changeable anastomosis device which can be deployed in the patient's body and enlarged and tightened without surgical interventions to at least temporarily hold anastomized tubular organs in tension and/or in compression. These and other objects are achieved by an adjustable shape changeable surgical implant system according to claim 1 Advantageous embodiments are claimed in the dependent claims. In accordance with an aspect of the invention, an adjustable shape changeable surgical implant system, e.g. e gastric band system, comprises:
- a swelling body suitable to absorb and release a fluid in order to swell and shrink respectively, thereby changing the shape of said implant,
- a selective receptor anchored inside said swelling body and configured to selectively bind a corresponding selective ligand,
- a shape control substance which can be administered to a patient having said implant, wherein the shape control substance comprises the selective ligand as well as a hydrophilic substance connected to the ligand, such that in response to the selective receptor binding the selective ligand, theswelling body absorbs liquid through hydrophilization, thereby changing the shape of the implant.

For better understanding the invention and appreciating the related advantages, a detailed and non limiting description of embodiments is provided with reference to the accompanying drawings, in which:
- Figure 1 is a schematic side view of a shape changeable gastric band in accordance with an embodiment of the invention,
- Figure 2 is a magnified view of a detail of a swelling body of the gastric band in figure 1,
- Figure 3 is a schematic representation of a selective receptor anchored to the swelling body wall in figure 2 and a shape control substance having a selective ligand and a hydrophilic substance connected thereto,
- Figure 4 is a schematic illustration of the shape changeable gastric band applied to the stomach of a patient,
- Figures 5 and 6 illustrate a shape changeable anastomotic ring device applied to an intestinal anastomosis site according to an embodiment of the invention,
- Figures 7 and 8 illustrate a shape changeable incontinence device applied to a rectum according to an embodiment of the invention,

Fig. 9 illustrates a shape adjustable bulking agent implanted in a patient's urinary tract to treat urinary incontinency according to an embodiment of the invention,

Fig. 10 illustrates an adjustable drug delivery device implanted in a patient's body according to an embodiment of the invention.

Turning to the figures, a shape changeable surgical implant system comprises a shape changeable surgical implant 1 having a swelling body 2 adapted to absorb and release a fluid in order to swell and shrink, respectively, thereby changing the shape of the surgical implant 1, as well as a selective receptor 3 anchored inside the swelling body 2 and configured to selectively bind a corresponding selective ligand 4.

A shape control substance 5 which can be administered, e.g. orally, to a patient carrying said implant 1, contains the above said selective ligand 4 as well as a hydrophilic substance 6 bond to the selective ligand 4. In this way, when the shape control substance 5 is administered to the patient, the selective receptor 3 of the swelling body 2 binds an amount of the selective ligand 4 together with the hydrophilic substance 6 dependent on the concentration of the shape control substance 5 in the patient's organism, so that the swelling body 2 absorbs body liquid through hydrophilization and swells, thereby changing the shape of said surgical implant 1.

Since the liquid absorption and swelling of the swelling body 2 is dependent from or approximately directly proportional with the concentration of the shape control substance 5 in the patient's body, a decrease of the concentration of the shape control substance 5 in the organism leads to a corresponding release of liquid from the swelling body 2 which will shrink, thereby reversely changing the shape of said surgical implant 1.

Accordingly, the shape changeable surgical implant system of the present invention makes it possible to control and adjust the shape of the surgical implant 2 through the dosage of the shape control substance 5 administered, e.g. orally, to the patient.

Throughout the following description, the expression "shape control substance" is intended to indicate a preferably non-pharmacologically active compound.

The shape of the swelling body 2 and its connection to or integration in the surgical implant 1 are configured in a manner that the substance 5 controlled adjustable swelling and shrinkage of the swelling body 2 results in the desired, pcssibly reversible and repeatable, overall or localized shape changes of the surgical implant 1.

In accordance with an embodiment, the swelling body 2 comprises a liquid permeable polymer matrix material 7 through which bodily fluids can permeate to make the swelling body 2 swell and shrink. Such a polymer matrix material 7 may have a cavernous, layered or fibrous microstructure which permits a high level of liquid diffusion and is preferably elastically or viscous-elastically deformable and non-biodegradable.

Non limiting examples of preferred liquid permeable polymer matrix materials 7 include:
- soft contact material Poly(2-hydroxyethyl methacrylate),
- nylon
- polyurethane.

In accordance with an embodiment, the selective receptor 3 is covalently bonded to the liquid permeable polymer matrix materials 7 of the swelling body 2 and specifically engineered or selected to only receive and bind the selective ligand 4.

In accordance with an embodiment, the selective ligand 4 of the shape control substance 5 comprises a synthetic biologically inert ligand which has only little or no effect on the patient's organism, but which will selectively bind to the receptor 3 located and strongly anchored (covalently bonded) in the swelling body 2.

The shape control substance 5 comprises preferably molecules having a ligand head forming the selective ligand 4 and a hydrophilic tail forming the hydrophilic substance 6, so that the ligand head binds to the selective receptors 3 of the swelling body 2 while the hydrophilic tail attracts water which causes the swelling body 2 to swell.

The distribution of the shape control substance 5 in the patient's organism and its elimination therefrom is governed by traditional pharmacokinetics and the shape control substance 5 must be assumed in regular intervals in order to maintain a constant shape control substance concentration in the patient's body and, hence, a constant shape configuration of the surgical implant 1. Augmentation and reduction of the shape control substance 5 dosage and administration frequency results in a corresponding shape variation of implant 1 due to hydrophilic swelling and dehydration shrinkage of the swelling body 2.

In accordance with an alternative embodiment, the hydrophilic substance 6 is replaced by a hydrophobic substance, so that administering the shape control substance 5 to a patient having the surgical implant 1 leads to hydrophobic shrinkage of the swelling body 2 and a subsequent decrease of the shape control substance 5 concentration in the patient's body brings about a reverse swelling of the swelling body 2 due to an equilibrium-absorption of bodily fluids.

In accordance with a yet further embodiment, an additional shape control substance (not specifically indicated in the figures) can be provided, which can be administered, e.g. orally, to a patient carrying said implant 1, and which contains the above said selective ligand as well as a hydrophobic substance bound to the selective ligand. In this way, when the additional shape control substance is administered to the patient, the selective receptor 3 of the swelling body 2 binds an amount of the selective ligand together with the hydrophobic substance dependent from the concentration of the additional shape control substance in the patient's organism, so that the swelling body 2 expels body fluids through hydrophobic action, thereby changing the shape of said surgical implant 1.

In accordance with a yet alternative embodiment, the hydrophilic substance 6 can be replaced by a pH sensitive substance adapted to attract H⁺ or OH⁻ ions, thereby causing shrinkage or swelling of the swelling body (2) in the presence of an acidic or basic environment, respectively. Examples of such pH sensitive substances are Ghitosan and bovine serum albumin which may be used to make a polymer matrix of the swelling body 2 pH sensitive. , so that administering the shape control substance 5 to a patient having the surgical implant 1 leads to hydrophobic shrinkage of the swelling body 2 and a subsequent decrease of the shape control substance 5 concentration in the patient's body brings about a reverse swelling of the swelling body 2 due to an equilibrium-absorption of bodily fluids.

In accordance with an embodiment, the selective receptor 3 and the selective ligand 4 comprise synthetic receptor/ligand pairs of the RASSL (Receptors Solely Activated by Synthetic Ligants) type, e.g. engineered G-proteins.

Non limiting examples for a receptor 3 / ligand 4 pair are:
1) A receptor comprising a hydrogel polymer modified with heparin-binding peptide and a ligand comprising a chemical compound including heparin.
2) A receptor comprising a cyclodextrin hydrogel polymer and a ligand comprising estradiol.

The covalent bound between the polymer matrix material 7 of the swelling body 2 and the selective receptor 3 can be obtained by the above indicated receptors 3, e.g. hydrogel and heparin binding peptide derived from antithombin III or estradiol/cyclodextrein hydrogel polymer.

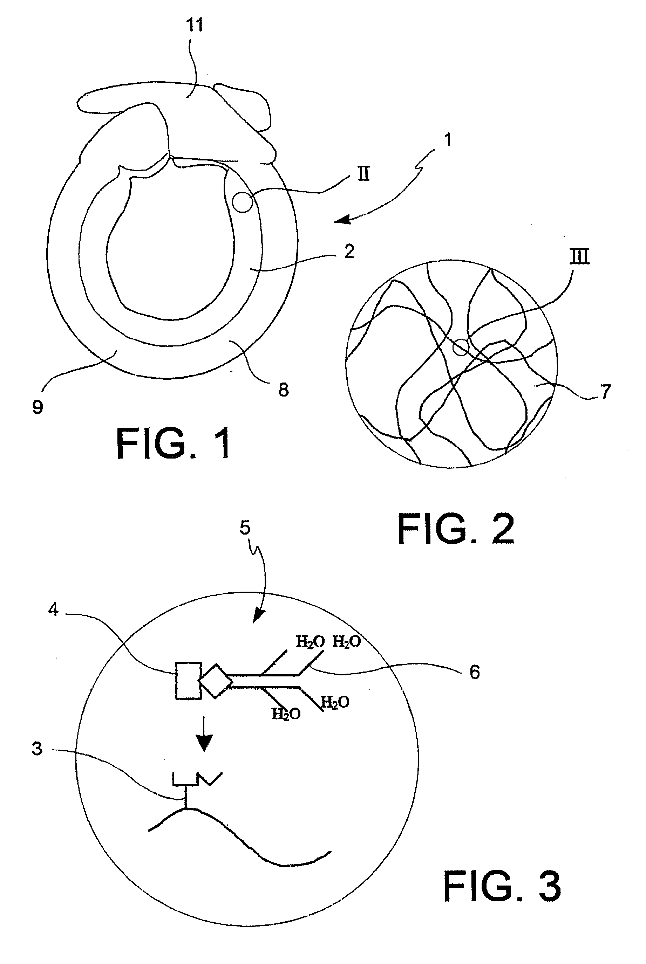

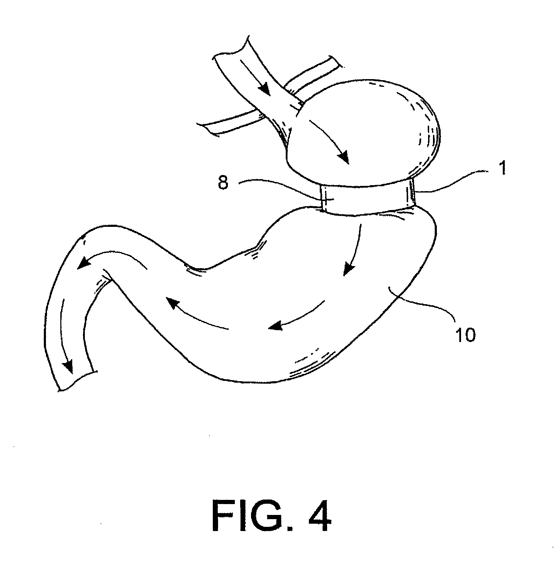

With reference to the figures 1 to 4, the surgical implant 1 may be embodied as a gastric band 8 having an elongate substantially non extensible tightening belt 9 which can be wound around the stomach 10 to form a strangling ring and locked by a closure device 11, wherein the swelling body 2 is connected to an internal side of the tightening belt 9 intended to face the gastric wall when the gastric band 8 is applied to stomach 10.

When administering the shape control substance 5 to the patient, the concentration of the shape control substance 5 in the patients organism increases and the selective ligands 4 bind to the selective receptors 3 in the swelling body 2 of the gastric band 8. The hydrophilic substance 6 bound to the ligands 4 and concentrated in the polymer matrix material 7 of the swelling body 2, attracts water which causes the swelling body 2 to swell, thereby reducing the internal diameter of the gastric band 8 and more intensely strangling the stomach 10 of the patient.

On interruption of the shape control substance 5 administering or dosage reduction, the concentration of the shape control substance 5 in the organism decreases due to physiological elimination of the shape control substance. This leads to a proportional reduction of the concentration or complete elimination of the shape control substance 5 in the swelling body 2 causing it to shrink due to an at least partial dehydration. Consequently, the internal diameter of the gastric band 8 increases and the stomach 10 of the patient becomes less intensely strangled.

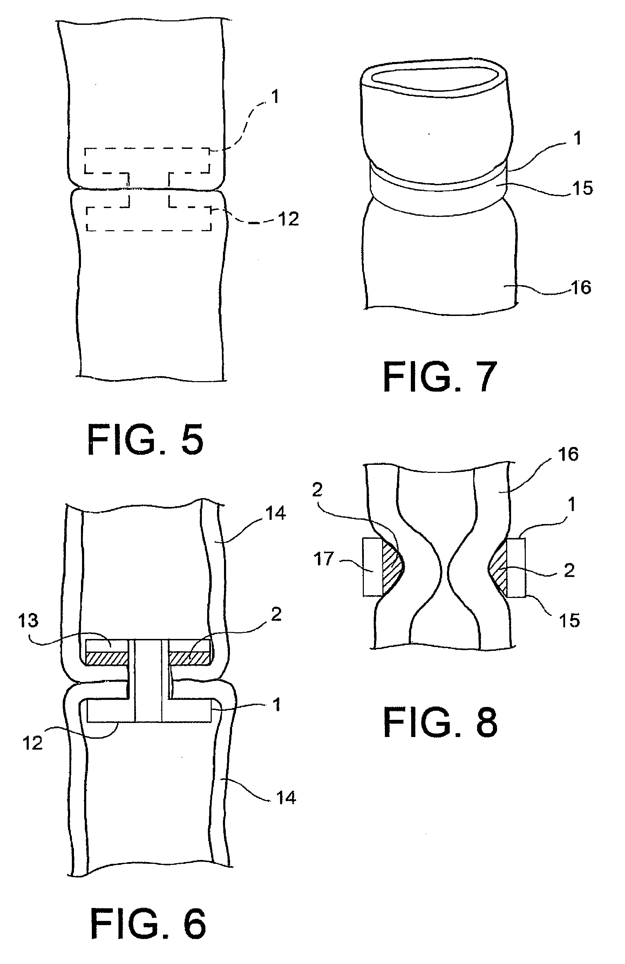

Figs. 5 and 6 illustrate an embodiment of the present invention, in which the surgical implant 1 comprises an anastomosis device, particularly an anastomotic ring device 12 with a substantially rigid ring body 13 which can connect two tissue portions of hollow organs (e.g. portions of an intestine 14) to join them in anastomosis, wherein the swelling body 2 is connected to or formed at a tissue contacting side of the ring body 13 to adjustably increase and release pressure on the tissue portions involved in the anastomosis by administering the shape control substance 5.

The mechanism of action is analogous to that described in relation with the previous embodiments.

Figs. 7 and 8 illustrate an embodiment of the present invention, in which the surgical implant 1 comprises a fecal or urinary incontinence device 15 which can be fastened around a patient's rectum 16 or urinary tract to control the passage of faeces and urine, respectively. The incontinence device may comprise a preferably inextensible base body 17 and the swelling body 2 is connected to or formed at a tissue contacting side of the base body 17 to adjustably strangle the rectum 16 or urinal tract in response to a controlled administering of the shape control substance 5. The mechanism of action is analogous to that described in relation with the previous embodiments.

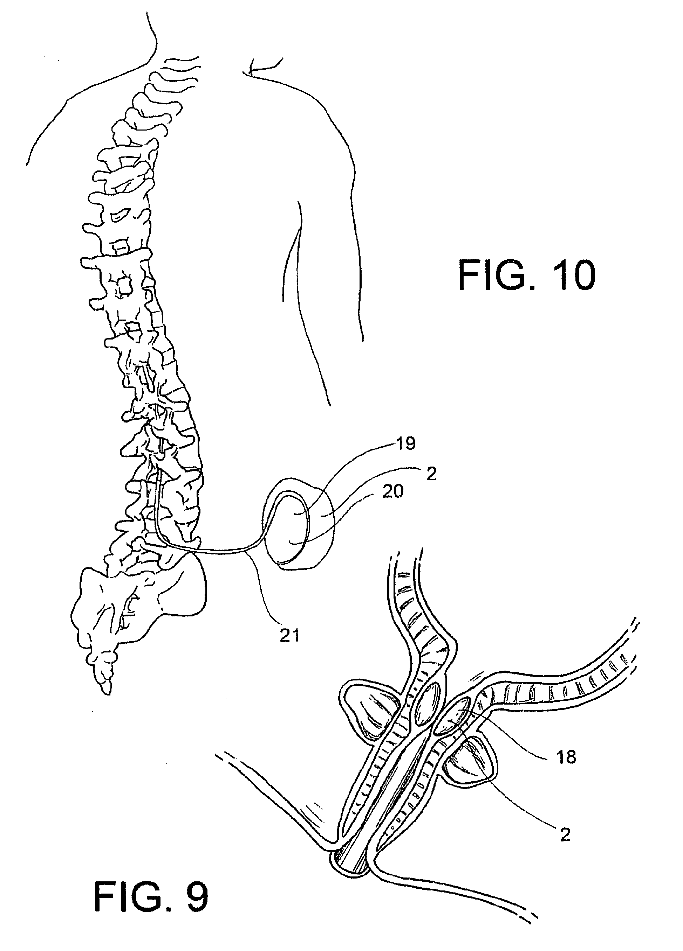

Fig. 9 illustrates a yet further embodiment, in which the surgical implant 1 comprises a shape adjustable bulking agent 18 which can be implanted in a patient's organs, e.g. lips, buttocks, fecal and urinary tracks, said bulking agent comprises the swelling body 2, preferably made of polymer or hydrogel material, adapted to adjust the bulkiness of the bulking agent 18 in response to a controlled administering of the shape control substance 5. The mechanism of action is analogous to that described in relation with the previous embodiments. The effect of the change of bulkiness of the bulking agent 18 can be aesthetic or therapeutic.

Fig. 10 illustrates a yet further embodiment, in which the surgical implant 1 comprises an adjustable drug delivery device 19 which can be implanted in a patient's body and comprises a drug reservoir 20 with drug dispensing duct 21, as well as the swelling body 2, preferably made of polymer or hydrogel material, connected to the drug reservoir 20 and adapted to stimulate or limit the release of a drug from the delivery device 19 in response to a controlled administering of the shape control substance 5. The mechanism of action is analogous to that described in relation with the previous embodiments.

The system according to the present invention reduces or avoids a patient's exposure to implant adjustments using a needle or surgery, so that the patient need not see the surgeon for implant adjustment but can adjust the implant shape by orally taking a specifically prescribed dosage of the shape control substance 5.

The system according to the invention provides an easily changeable and customizable implant shape and size suitable for individually different patients.

The system allows a nearly unlimited and relatively quick implant shape adjustment, e.g. gastric band pressure adjustment, and enables the patient to gain more control over the treatment.

Although preferred embodiments of the invention have been described in detail, it is not the intention of the applicant to limit the scope of the claims to such particular embodiments, but to cover all modifications and alternative constructions falling within the scope of the invention.

## Claims

1. An adjustable shape changeable surgical implant system, comprising:
- a shape changeable surgical implant (1) having a swelling body (2) adapted to absorb and release fluid to swell and shrink, respectively, thereby changing the shape of said implant (1),
- a selective receptor (3) anchored inside said swelling body (2) and configured to selectively bind a corresponding selective ligand (4),
- a shape control substance (5) which can be administered to a patient having said shape changeable surgical implant (1), wherein said shape control substance (5) comprises said selective ligand (4) as well as a hydrophilic substance (6) connected to said ligand (4), such that in response to said selective receptor (3) binding said selective ligand (4), said swelling body absorbs liquid through hydrophilization, thereby changing the shape of said implant (1).

2. A system according to claim 1, in which the fluid absorption and swelling of the swelling body (2) is dependent from the concentration of said shape control substance (5) in the patient's body, such that a decrease of the concentration of the shape control substance (5) in the organism of said patient leads to a corresponding release of liquid from the swelling body (2) which will shrink, thereby reversely changing the shape of said surgical implant (1).

3. A system according to claim 1 or 2, in which said shape control substance (5) is configured to be administered orally.

4. A system according to any one of the preceding claims, in which said swelling body (2) comprises a liquid permeable polymer matrix material (7) through which bodily fluids can permeate to make the swelling body (2) swell and shrink.

5. A system according to claim 4, in which said polymer matrix material (7) is selected in the group comprising Poly(2-hydroxyethyl methacrylate), nylon, polyurethane.

6. A system according to any one of the preceding claims, in which said selective receptor (3) is covalently bonded to said swelling body (2) and specifically engineered to only receive and bind the selective ligand (4).

7. A system according to any one of the preceding claims, in which said selective ligand (4) of the shape control substance (5) comprises a synthetic biologically inert ligand to avoid side effects on the human organism.

8. A system according to any one of the preceding claims, comprising an additional shape control substance which can be administered to the patient carrying said implant (1), said additional shape control substance containing said selective ligand (4) and a hydrophobic substance bond to said selective ligand (4) and adapted to cause hydrophobic shrinkage of said swelling body (2), thereby changing the shape of said surgical implant (1).

9. A system according to any one of claims 1 to 7, in which the hydrophilic substance (6) is replaced by a substance selected in the group consisting of:
- hydrophobic substances adapted to cause hydrophobic shrinkage of the swelling body (2),
- pH sensitive substances adapted to attract H⁺ or OH⁻ ions, thereby causing shrinkage or swelling of the swelling body (2) in the presence of an acidic or basic environment, respectively.

10. A system according to any one of the preceding claims, in which said selective receptor (3) and said selective ligand (4) comprise synthetic receptor/ligand pairs of the RASSL (Receptors Solely Activated by Synthetic Ligants) type.

11. A system according to any one of claims 1 to 10, in which said surgical implant (1) comprises a gastric band (8) with a tightening belt (9) which can be wound around a stomach (10) to form a strangling ring, wherein the swelling body (2) is connected to said tightening belt (9) to adjustably increase and release pressure on the stomach (10) in response to administering said shape control substance (5).

12. A system according to any one of claims 1 to 10, in which said surgical implant (1) comprises an anastomosis device with a ring body (13) adapted to connect two tissue portions of hollow organs to join them in anastomosis, wherein said swelling body (2) is connected to said ring body (13) to adjustably increase and release pressure on said tissue portions in response to administering said shape control substance (5).

13. A system according to any one of claims 1 to 10, in which said surgical implant (1) comprises an incontinence device (15) which can be fastened around a patient's rectum (16) or urinary tract to control the passage of faeces and urine, respectively, said incontinence device (15) comprising a base body (17) and said swelling body (2) is connected to said base body (17) to adjustably strangle the rectum (16) or urinal tract in response to administering the shape control substance (5).

14. A system according to any one of claims 1 to 10, in which said surgical implant (1) comprises an adjustable bulking agent (18).

15. A system according to any one of claims 1 to 10, in which said surgical implant (1) comprises an adjustable drug delivery device (19).

## Patentansprüche

1. Einstellbares formveränderliches chirurgisches Implantationssystem, das Folgendes umfasst:
- ein formveränderliches chirurgisches Implantat (1) mit einem Anschwellkörper (2), der dazu eingerichtet ist, ein Fluid zu absorbieren und freizugeben, um anzuschwellen bzw. zu schrumpfen, wodurch sich die Form des Implantats (1) ändert,
- einen selektiven Rezeptor (3), der innerhalb des Anschwellkörpers (2) veran-kert ist und dazu konfiguriert ist, selektiv einen entsprechenden selektiven Liganden (4) zu binden,
- eine Formsteuerungssubstanz (5), die einem Patienten, der das formveränderliche chirurgische Implantat (1) hat, verabreicht werden kann, wobei die Formsteuerungssubstanz (5) den selektiven Liganden (4) sowie eine hydrophile Substanz (6), die mit dem Liganden (4) verbunden ist, umfasst, so dass der Anschwellkörper in Antwort darauf, dass der selektive Rezeptor (3) den selektiven Liganden (4) bindet, eine Flüssigkeit durch Hydrophilisierung absorbiert, wodurch sich die Form des Implantats (1) verändert.

2. System gemäß Anspruch 1, bei dem die Fluidabsorption und das Anschwellen des Anschwellkörpers (2) von der Konzentration der Formsteuerungssubstanz (5) in dem Körper des Patienten abhängt, so dass eine Abnahme der Konzentration der Formsteuerungssubstanz (5) in dem Organismus des Patienten zu einer entsprechenden Freigabe einer Flüssigkeit aus dem Anschwellkörper (2), der schrumpfen wird, führt, wodurch sich die Form des chirurgischen Implantats (1) in die umgekehrte Richtung verändert.

3. System gemäß Anspruch 1 oder 2, bei dem die Formsteuerungssubstanz (5) dazu konfiguriert ist, oral verabreicht zu werden.

4. System gemäß einem der vorhergehenden Ansprüche, bei dem der Anschwellkörper (2) ein flüssigkeitspermeables Polymermatrixmaterial (7), durch welches Körperflüssigkeiten dringen können, umfasst, um den Anschwellkörper (2) zum Anschwellen und Schrumpfen zu bringen.

5. System gemäß Anspruch 4, bei dem das Polymermatrixmaterial (7) ausgewählt ist aus der Gruppe, die Poly(2-Hydroxyethylmethacrylat), Nylon und Polyurethan umfasst.

6. System gemäß einem der vorhergehenden Ansprüche, bei dem der selektive Rezeptor (3) kovalent an den Anschwellkörper (2) gebunden ist und im Besonderen dazu ausgelegt ist, nur den selektiven Liganden (4) aufzunehmen und zu binden.

7. System gemäß einem der vorhergehenden Ansprüche, bei dem der selektive Ligand (4) der Formsteuerungssubstanz (5) einen synthetischen biologisch inerten Liganden umfasst, um Nebenwirkungen auf den menschlichen Organismus zu vermeiden.

8. System gemäß einem der vorhergehenden Ansprüche, das eine zusätzliche Formsteuerungssubstanz umfasst, die dem Patienten, der das Implantat (1) trägt, verabreicht werden kann, wobei die zusätzliche Formsteuerungssubstanz den selektiven Liganden (4) und eine hydrophobe Substanz, die an den selektiven Liganden (4) gebunden ist und dazu ausgelegt ist, ein hydrophobes Schrumpfen des Anschwellkörpers (2) zu bewirken, wodurch sich die Form des chirurgischen Implantats (1) verändert, enthält.

9. System gemäß einem der Ansprüche 1 bis 7, bei dem die hydrophile Substanz (6) durch eine Substanz ersetzt ist, die aus einer Gruppe ausgewählt ist, die aus Folgendem besteht:
- hydrophoben Substanzen, die dazu ausgelegt sind, ein hydrophobes Schrump-fen des Anschwellkörpers (2) zu bewirken,
- pH-empfindlichen Substanzen, die dazu ausgelegt sind, H+- oder OH--Ionen anzuziehen, wodurch ein Schrumpfen oder ein Anschwellen des Anschwell-körpers (2) in der Gegenwart einer sauren bzw. einer basischen Umgebung be-wirkt wird.

10. System gemäß einem der vorhergehenden Ansprüche, bei dem der selektive Rezeptor (3) und der selektive Ligand (4) synthetische Rezeptor-/Ligandenpaare des RASSL (Receptors Solely Activated by Synthetic Ligands)-Typs umfassen.

11. System gemäß einem der Ansprüche 1 bis 10, bei dem das chirurgische Implantat (1) ein Magenband (8) mit einem Straffungsgurt (9) umfasst, das um einen Magen (10) gewickelt werden kann, um einen Würgering zu bilden, wobei der Anschwellkörper (2) mit dem Straffungsgurt (9) verbunden ist, um einen Druck auf den Magen (10) in Antwort auf ein Verabreichen der Formsteuerungssubstanz (5) einstellbar zu erhöhen und freizugeben.

12. System gemäß einem der Ansprüche 1 bis 10, bei dem das chirurgische Implantat (1) eine Anastomosevorrichtung mit einem Ringkörper (13) umfasst, die dazu ausgelegt ist, zwei Gewebeabschnitte von Hohlorganen zu verbinden, um sie in einer Anastomose zusammenzufügen, wobei der Anschwellkörper (2) mit dem Ringkörper (13) verbunden ist, um einen Druck auf die Gewebeabschnitte in Antwort auf ein Verabreichen der Formsteuerungssubstanz (5) einstellbar zu erhöhen und freizugeben.

13. System gemäß einem der Ansprüche 1 bis 10, bei dem das chirurgische Implantat (1) eine Inkontinenzvorrichtung (15) umfasst, die um ein Rektum (16) oder einen Harnorgantrakt eines Patienten herum angebracht werden kann, um den Durchgang von Kot bzw. Urin zu steuern, wobei die Inkontinenzvorrichtung (15) einen Basiskörper (17) umfasst, und wobei der Anschwellkörper (2) mit dem Basiskörper (17) verbunden ist, um das Rektum (16) oder den Harnorgantrakt in Antwort auf ein Verabreichen der Formsteuerungssubstanz (5) einstellbar zu würgen.

14. System gemäß einem der Ansprüche 1 bis 10, bei dem das chirurgische Implantat (1) ein einstellbares Quellmittel (18) umfasst.

15. System gemäß einem der Ansprüche 1 bis 10, bei dem das chirurgische Implantat (1) eine einstellbare Arzneimittelverabreichungsvorrichtung (19) umfasst.

## Revendications

1. Système d'implant chirurgical à changement de forme ajustable, comprenant :
- un implant chirurgical à changement de forme (1) comportant un corps pouvant gonfler (2) adapté pour absorber et libérer un fluide pour gonfler et se rétracter, respectivement, en modifiant ainsi la forme dudit implant (1),
- un récepteur sélectif (3) ancré à l'intérieur dudit corps pouvant gonfler (2) et configuré pour se lier sélectivement à un ligand sélectif (4) correspondant,
- une substance de contrôle de la forme (5) qui peut être administrée à un patient portant ledit implant chirurgical à changement de forme (1), dans lequel ladite substance de contrôle de la forme (5) comprend ledit ligand sélectif (4) ainsi qu'une substance hydrophile (6) reliée audit ligand (4), de sorte qu'en réponse à la liaison dudit récepteur sélectif (3) audit ligand sélectif (4), ledit corps pouvant gonfler absorbe le liquide par hydrophilisation, en modifiant ainsi la forme dudit implant (1).

2. Système selon la revendication 1, dans lequel l'absorption du fluide et le gonflement du corps pouvant gonfler (2) dépendent de la concentration de ladite substance de contrôle de la forme (5) dans le corps du patient, de sorte qu'une diminution de la concentration de la substance de contrôle de la forme (5) dans l'organisme dudit patient entraîne une libération correspondante de liquide du corps pouvant gonfler (2) qui se rétracte, en changeant ainsi inversement la forme dudit implant chirurgical (1).

3. Système selon la revendication 1 ou 2, dans lequel ladite substance de contrôle de la forme (5) est configurée pour être administrée par voie orale.

4. Système selon l'une quelconque des revendications précédentes, dans lequel ledit corps pouvant gonfler (2) comprend un matériau à matrice polymère perméable aux liquides (7) dans lequel les fluides corporels peuvent pénétrer pour que le corps pouvant gonfler (2) se gonfle et se rétracte.

5. Système selon la revendication 4, dans lequel ledit matériau à matrice polymère (7) est choisi dans le groupe comprenant le poly(méthacrylate de 2-hydroxyéthyle), le nylon, le polyuréthane.

6. Système selon l'une quelconque des revendications précédentes, dans lequel ledit récepteur sélectif (3) est lié par covalence audit corps pouvant gonfler (2) et est spécifiquement conçu pour uniquement recevoir le ligand sélectif (4) et s'y lier.

7. Système selon l'une quelconque des revendications précédentes, dans lequel ledit ligand sélectif (4) de la substance de contrôle de la forme (5) comprend un ligand synthétique biologiquement inerte pour éviter les effets secondaires sur l'organisme humain.

8. Système selon l'une quelconque des revendications précédentes, comprenant une substance de contrôle de la forme additionnelle qui peut être administrée au patient portant ledit implant (1), ladite substance de contrôle de la forme additionnelle contenant ledit ligand sélectif (4) et une substance hydrophobe liée audit ligand sélectif (4) et adaptée pour provoquer le retrait hydrophobe dudit corps pouvant gonfler (2), en modifiant ainsi la forme dudit implant chirurgical (1).

9. Système selon l'une quelconque des revendications 1 à 7, dans lequel la substance hydrophile (6) est remplacée par une substance choisie dans le groupe constitué :
- des substances hydrophobes adaptées pour provoquer le retrait hydrophobe du corps pouvant gonfler (2),
- des substances sensibles au pH adaptées pour attirer les ions H+ ou OH-, en provoquant ainsi le retrait ou le gonflement du corps pouvant gonfler (2) en présence d'un environnement acide ou basique, respectivement.

10. Système selon l'une quelconque des revendications précédentes, dans lequel ledit récepteur sélectif (3) et ledit ligand sélectif (4) comprennent des paires récepteur/ligand synthétiques de type RASSL (récepteurs uniquement activés par des ligands synthétiques).

11. Système selon l'une quelconque des revendications 1 à 10, dans lequel ledit implant chirurgical (1) comprend un anneau gastrique (8) doté d'une ceinture de serrage (9) qui peut être enroulée autour d'un estomac (10) pour former un anneau de strangulation, dans lequel le corps pouvant gonfler (2) est relié à ladite ceinture de serrage (9) pour augmenter et relâcher de manière ajustable la pression sur l'estomac (10) en réponse à l'administration de ladite substance de contrôle de la forme (5).

12. Système selon l'une quelconque des revendications 1 à 10, dans lequel ledit implant chirurgical (1) comprend un dispositif d'anastomose doté d'un corps annulaire (13) adapté pour relier deux parties tissulaires d'organes creux pour les relier en anastomose, dans lequel ledit corps pouvant gonfler (2) est relié audit corps annulaire (13) pour augmenter et relâcher de manière ajustable la pression sur lesdites parties tissulaires en réponse à l'administration de ladite substance de contrôle de la forme (5).

13. Système selon l'une quelconque des revendications 1 à 10, dans lequel ledit implant chirurgical (1) comprend un dispositif pour l'incontinence (15) qui peut être fixé autour du rectum (16) ou du tractus urinaire d'un patient pour contrôler le passage des selles et des urines, respectivement, ledit dispositif pour l'incontinence (15) comprenant un corps de base (17) et ledit corps pouvant gonfler (2) est relié audit corps de base (17) pour étrangler de manière ajustable le rectum (16) ou le tractus urinaire en réponse à l'administration de la substance de contrôle de la forme (5).

14. Système selon l'une quelconque des revendications 1 à 10, dans lequel ledit implant chirurgical (1) comprend un agent gonflant ajustable (18).

15. Système selon l'une quelconque des revendications 1 à 10, dans lequel ledit implant chirurgical (1) comprend un dispositif d'administration de médicaments ajustable (19).
